# EUROPÄISCHE PATENTSCHRIFT

(11) **EP 1 855 653 B1**
(45) Veröffentlichungstag und Bekanntmachung des Hinweises auf die Patenterteilung: **15.08.2012**
(21) Anmeldenummer: 06723283.5
(22) Anmeldetag: 08.03.2006
(51) Int. Cl.: A61K 9/16

(54) **ZUSAMMENSETZUNG FÜR DIE PERORALE APPLIKATION MIT GESTEUERTER FREISETZUNG VON WIRKSTOFFEN**
COMPOSITION FOR ORAL APPLICATION WITH CONTROLLED RELEASE OF ACTIVE SUBSTANCES
COMPOSITION POUR ADMINISTRATION ORALE A LIBERATION CONTROLEE DE PRINCIPES ACTIFS

(30) Priorität: 08.03.2005 DE 102005011029
(43) Veröffentlichungstag der Anmeldung: 21.11.2007
(73) Patentinhaber: Maria Clementine Martin Klosterfrau Vertriebsgesellschaft mbH, 50670 Köln (DE)
(72) Erfinder: MIETHING, Holger, 12107 Berlin (DE)
(74) Vertreter: Gesthuysen, von Rohr & Eggert
(86) Internationale Anmeldenummer: PCT/EP2006/002122
(87) Internationale Veröffentlichungsnummer: WO 2006/094782

(56) Entgegenhaltungen:
- EP-A- 0 250 187
- EP-A- 0 497 977
- WO-A-01/72286
- WO-A-97/47285
- WO-A-99/45887
- GB-A- 2 163 648

## Beschreibung

Die vorliegende Erfindung betrifft eine Zusammensetzung für die perorale Applikation mit gesteuerter Freisetzung von Wirkstoffen.

Insbesondere betrifft die vorliegende Erfindung eine Zusammensetzung für die perorale Applikation mit gesteuerter Freisetzung von Wirkstoffen aus der Gruppe von Vitaminen, Mineralstoffen und Spurenelementen sowie deren Verwendung.

Bei vielen Wirkstoffen ist eine kontrollierte bzw. retardierte Freisetzung nach ihrer peroralen Applikation gewünscht. Dies ist insbesondere dann der Fall, wenn Kombinationen von Wirkstoffen appliziert werden, welche sich in ihrer Wirkweise untereinander beeinflussen können.

So ist beispielsweise bekannt, daß verschiedene Vitalstoffe - hierunter werden im Rahmen der vorliegenden Erfindung Vitamine, Mineralstoffe und Spurenelemente verstanden - sich bei gleichzeitiger Applikation bezüglich ihrer Resorption negativ beeinflussen können (z. B. Kupfer und Zink oder aber Magnesium und Calcium etc.).

Der Bedarf an Vitalstoffen kann nicht immer mit der täglichen Ernährung gedeckt werden. Ursache dafür ist unter anderem, daß ein Großteil der in Lebensmitteln enthaltenen Vitalstoffe (d. h. Vitamine, Mineralstoffe und Spurenelemente) durch Überlagerung oder aber durch falsche Zubereitung, wie zu langes Kochen, Aufwärmen etc., verlorengehen. Beispielsweise wird der Gehalt an Vitamin B₆ in Milch durch Erhitzen oder durch längere Sonnenbestrahlung um bis zu 50 % reduziert.

Insbesondere ältere bzw. übergewichtige Menschen sowie Personen, die durch körperliche Arbeit oder sportliche Aktivitäten stark beansprucht sind, können einen erhöhten Bedarf an Vitalstoffen haben. Aber auch bei Menschen, die sich einseitig ernähren oder aber die eine Diät machen, kann es zu einer Unterversorgung an Vitalstoffen kommen.

Der menschliche Körper kann die Vitalstoffe weitgehend nicht selbst herstellen und nur begrenzt speichern. Deshalb müssen dem Organismus diese Vitalstoffe regelmäßig und in ausreichenden Mengen mit der Nahrung zugeführt werden.

Da dies mit der üblichen Nahrung nicht immer zu realisieren ist, werden Vitalstoffpräparate (z. B. Arzneimittel, Nahrungsergänzungsmittel, Medizinprodukte etc.) verabreicht, welche den Vitalstoffbedarf decken bzw. ergänzen sollen.

Ein Nachteil vieler handelsüblicher Präparate liegt aber darin, daß in vielen Fällen die Vitalstoffe nach peroraler Applikation unkontrolliert und in übermäßigen Mengen freigesetzt werden, so daß durch das schnelle Anfluten der Vitalstoffe die menschlichen Resorptionssysteme, insbesondere der Magen-/Darmtrakt, überlastet ist, so daß zum einen nur ein Teil der angebotenen Vitalstoffe aufgenommen werden kann und dies zum anderen nur über einen relativ kurzen Zeitraum erfolgt. Dies bedeutet, daß mit der ersten Aufnahme dann bereits im Körper der Verbrauch und die Ausscheidung der applizierten Vitalstoffe erfolgen.

Um diesem Problem zu begegnen, wurden bereits zahlreiche Versuche unternommen, die zu applizierenden Vitalstoffe in ein System zu inkorporieren, welches die Vitalstoffe nach peroraler Applikation kontrolliert freisetzt. Beispielsweise beschreibt die WO 01/72286 A1 (PCT/EP 01/03192) eine therapeutische Formulierung für die perorale Applikation von Vitalstoffkombinationen aus Vitaminen, Spurenelementen und Mineralstoffen, wobei die verzögerte bzw. kontrollierte Freisetzung durch spezielle Beschichtungen geeigneter Kapselsysteme erreicht werden soll, wobei derartige Beschichtungen beispielsweise magensaftresistent ausgebildet sein können (z. B. Beschichtungen auf Schellackbasis). Nachteil dieser Kapselsysteme ist einerseits ihre relativ aufwendige Herstellung und andererseits die geringe Variabilität in bezug auf die Freisetzungskinetik. Auch kann mit derartigen Kapselsystemen nicht immer der gewünschte Freisetzungseffekt in ausreichendem Maße realisiert werden.

Das der vorliegenden Erfindung zugrundeliegende Problem besteht somit darin, eine für die perorale Applikation bestimmte Zusammensetzung bzw. ein für die perorale Applikation bestimmtes System bereitzustellen, welche bzw. welches die darin enthaltenen Wirkstoffe nach peroraler Applikation gezielt bzw. in gesteuerter Weise freisetzt und insbesondere die vorgenannten Nachteile des Standes der Technik zumindest weitgehend vermeidet oder aber zumindest abschwächt.

Die Anmelderin hat nun überraschenderweise herausgefunden, daß das zuvor geschilderte, der vorliegenden Erfindung zugrundeliegende Problem dadurch gelöst werden kann, daß die Wirkstoffe zumindest teilweise in einer quellfähigen Matrix inkorporiert bzw. hierin eingelagert werden.

Gegenstand der vorliegenden Erfindung ist somit - gemäß einem ersten Aspekt - eine Zusammensetzung nach Anspruch 1. Weitere, vorteilhafte Ausgestaltungen sind Gegenstand der diesbeüglichen Unteransprüche.

Gegenstand der vorliegenden Erfindung ist somit - gemäß einem ersten Aspekt - eine Zusammensetzung für die perorale Applikation mit gesteuerter Freisetzung von Wirkstoffen, wobei die Zusammensetzung eine Vielzahl bzw. eine Kombination von Wirkstoffen enthält, welche insbesondere ausgewählt sind aus der Gruppe von Vitaminen, Mineralstoffen und Spurenelementen sowie deren Mischungen, wobei die Wirkstoffe zumindest teilweise in einer quellfähigen Matrix inkorporiert bzw. hierin eingelagert sind, so daß die Wirkstoffe nach peroraler Applikation kontrolliert und/oder zeitversetzt freigesetzt werden.

Die erfindungsgemäße, für die perorale Applikation vorgesehene Zusammensetzung - synonym auch gelegentlich als erfindungsgemäßes System bezeichnet - enthält somit eine Vielzahl bzw. eine Kombination verschiedener Wirkstoffe, insbesondere aus der Gruppe von Vitaminen, Mineralstoffen und/oder Spurenelementen, welche kontrolliert bzw. zeitlich versetzt zueinander, d. h. zu unterschiedlichen Zeitpunkten und/oder an unterschiedlichen Resorptionsorten (d. h. Magen, Duodenum bzw. oberer Dünndarm, restlicher Dünndarm, Darm bzw. Colon etc.), oder aber retardiert über einen verlängerten Zeitraum freigesetzt werden. Die Formulierung "kontrolliert und/oder zeitversetzt", wie sie im Rahmen der vorliegenden Erfindung verwendet wird, kann sowohl die Retardierung der Freisetzung als auch die zeitliche Versetzung der Freisetzung unterschiedlicher Wirkstoffe zueinander bezeichnen.

Da die erfindungsgemäße Zusammensetzung bzw. das erfindungsgemäße System eine kontrollierte und/oder zeitversetzte Freisetzung unterschiedlicher Wirkstoffe, welche in einer einzigen Kombination bzw. in einer einzigen Zusammensetzung vorliegen, ermöglicht, eignet sich die erfindungsgemäße Zusammensetzung bzw. das erfindungsgemäße System für die perorale Applikation insbesondere von Vitalstoffen (d. h. also Wirkstoffen, welche aus der Gruppe von Vitaminen, Mineralstoffen und Spurenelementen sowie deren Mischungen ausgewählt sind).

Unter dem Begriff der Vitamine werden im Rahmen der vorliegenden Erfindung insbesondere organische Substanzen verstanden, welche zur Aufrechterhaltung von Gesundheit und Leistungsfähigkeit des menschlichen Organismus notwendig sind und mit der Nahrung zugeführt werden müssen. Es genügen täglich wenige Milligramm, um die Verwertung der Nährstoffe (insbesondere Kohlenhydrate, Fette, Eiweiße und Mineralstoffe) zu regulieren. Jedes einzelne Vitamin erfüllt besondere Aufgaben, die von einem anderen Vitamin nicht in gleicher Weise ausgeübt werden können. Für vitaminähnliche Verbindungen, die noch nicht nachgewiesenermaßen essentiell für den Menschen sind, hat sich auch die Bezeichnung Vitaminoide eingebürgert. Von ihrer Nomenklatur her werden die Vitamine in verschiedene Gruppen eingeteilt, z. B. Vitamine der A-Gruppe (z. B. Vitamine A₁ und A₂), Vitamine der B-Gruppe (z. B. Vitamine B₁, B₂, B₆ und B₁₂, Biotin, Folsäure, Nicotinsäure bzw. Nicotinsäureamid, Pantothensäure etc.), Vitamine der D-Gruppe (z. B. Vitamine D₁, D₂ und D₃), Vitamine der E-Gruppe, Vitamin F, Vitamin G (frühere Bezeichnung für Riboflavin), Vitamin H (synonyme Bezeichnung für Biotin), Vitamin H', Vitamin I oder J, Vitamine der K-Gruppe, Vitamine der L-Gruppe, Vitamin M, Vitamin P, Vitamin Q, Vitamin T, Vitamin U etc. In bezug auf weitere Einzelheiten zu Vitaminen kann beispielsweise verwiesen werden auf Römpp Chemielexikon, 10. Auflage, Band 6, 1999, Georg Thieme Verlag Stuttgart/New York, Seiten 4877 bis 4887, Stichwort: "Vitamine", sowie die dort referierte Literatur.

Im Unterschied zu den Vitaminen wird der Begriff der Mineralstoffe als Sammelbezeichnung für die mineralischen Bestandteile der pflanzlichen und tierischen Organismen (beim Menschen ca. 3,5 kg) verwendet. Man unterscheidet sogenannten Mikroelemente mit Spurenelementcharakter, die hauptsächlich katalytische Funktionen ausüben (F, Br, I, Fe, Cu, Mn, Co, Zn, Mo, V, Se), und die mengenmäßig weit überwiegenden Makroelemente, die als Baustoffe (z. B. für das Skelett) unentbehrlich sind (Ca, Na, K, P, S, Mg, Cl). Außerdem unterscheidet man essentielle Mineralstoffe mit bekannter biologischer Funktion sowie akzidentelle Mineralstoffe mit (vorläufig noch) unbekannter Funktion. Bisher wurden vierundzwanzig chemische Elemente als essentielle (lebensnotwendige) Wirkstoffe für den menschlichen Organismus erkannt. Die Mineralstoffe werden mit den Nährstoffen in den Organismus aufgenommen. Sie werden zu einem großen Teil durch die natürlichen Ausscheidungen (z. B. Kot, Harn, Schweiß) wieder abgegeben und müssen daher stetig ergänzt werden. Auf diese Weise entsteht ein ausgeprägter Mineralstoffkreislauf (Mineralstoffwechsel). In bezug auf weitere Einzelheiten zu dem Begriff der Mineralstoffe kann beispielsweise verwiesen werden auf Römpp Chemielexikon, 10. Auflage, Band 4, 1998, Georg Thieme Verlag Stuttgart/New York, Seiten 2700/2701, Stichwort: "Mineralstoffe", sowie die dort referierte Literatur.

Schließlich bezeichnet der Begriff der Spurenelemente eine Reihe von chemischen Elementen, welche der menschliche Organismus nur in Spuren enthält, die aber oftmals lebenswichtige Aufgaben erfüllen. Die vorgeschlagene Größenordnung für lebensnotwendige Mineralstoffe hinsichtlich ihrer Zuordnung zur Gruppe der Spurenelemente liegt bei Werten unter 50 mg/kg bezüglich der Konzentration im Organismus und in der Nahrung. Demgegenüber liegt der Körperbestand an den zu den Mengenelementen (Makroelementen) zu zählenden Mineralstoffen Ca, P, K, Cl, Na und Mg mit teilweise über 1 g/kg deutlich darüber. Die Konzentration für essentielle Spurenelemente im menschlichen Organismus liegt zwischen 0,02 bis 60 mg/kg, für nichtessentielle Spurenelemente zwischen 0,0004 bis 4,6 mg/kg. Die Tageszufuhr an Spurenelementen bewegt sich in Abhängigkeit von der Nahrung etwa zwischen 0,002 bis 32 mg. Über die Verteilung im_Körper liegen ebenfalls Werte vor. Ihre Wirkung entfalten die Spurenelemente nur in ionisch gelöster oder komplexgebundener Form. Eine Einteilung der Spurenelemente kann wie folgt geschehen: Erstens: Elemente, deren Notwendigkeit für den Menschen erwiesen ist und deren Mangel zur Manifestation klinischer Symptome führt (sogenannte essentielle Spurenelemente, z. B. Eisen, Kupfer, Zink, Chrom, Selen, Lithium, Cobalt, Molybdän, Iod, Silicium, Fluor und Mangan); zweitens: Elemente deren Funktion für den Menschen noch nicht genügend gesichert ist (z. B. Zinn, Nickel, Vanadium und Arsen); drittens: Elemente, die in größeren Mengen beim Menschen toxisch wirken (z. B. Cadmium, Molybdän, Blei, Quecksilber, Arsen, Bor und Zinn). Im Rahmen dieser Einteilung, die wie das Beispiel Molybdän oder Zinn zeigt, fließend ist, ist zu bedenken, daß auch essentielle Spurenelemente in höheren Konzentrationen toxisch sein können (z. B. Cobalt) und daß bisher als toxisch eingestufte Spurenelemente später in geringerer Konzentration als essentiell erkannt werden können (z: B. Selen, das bis 1957 ausschließlich als toxisches Spurenelement betrachtet wurde, aber von dem heute bekannt ist, daß es in verschiedenen Enzymen, z. B. Glutathion-Peroxidase etc., vorkommt). Für weitere Einzelheiten zu dem Begriff der Spurenelemente kann beispielsweise verwiesen werden auf Römpp Chemielexikon, 10. Auflage, Band 5, 1998, Georg Thieme Verlag Stuttgart/New York, Seiten 4202 bis 4204, Stichwort: "Spurenelemente", sowie die dort referierte Literatur.

Im Rahmen der erfindungsgemäßen Zusammensetzung sind die Wirkstoffe, insbesondere Vitamine, Mineralstoffe und/oder Spurenelemente, zumindest teilweise in einer quellfähigen Matrix inkorporiert bzw. eingelagert.

Der Begriff der Matrix bezeichnet im Rahmen der vorliegenden Erfindung ein in bezug auf die einzulagernden Wirkstoffe zumindest im wesentlichen inertes Hüllmaterial, welches die Wirkstoffe zunächst eingeschlossen enthält. Das Matrixmaterial ist derart ausgewählt, daß es quellfähig ist, d. h. insbesondere bei Kontakt mit wäßrigen fluiden Medien, insbesondere bei Kontakt mit Magen- und/oder Darmsaft, quellfähig ist bzw. aufquillt. Auf diese Weise wird eine Diffusionsbarriere bzw. eine mechanische Barriere für die in die Matrix einzulagernden und nachfolgend kontrolliert bzw. zeitversetzt freizusetzenden Wirkstoffe ausgebildet, so daß die Wirkstoffe nach peroraler Applikation nicht spontan entweichen können, sondern kontrolliert bzw. retardiert freigesetzt werden (z. B. durch Diffusionsprozesse im Magen-/Darmtrakt).

Die Formulierung, daß die Wirkstoffe "zumindest teilweise" in die quellfähige Matrix inkorporiert bzw. eingelagert sind, meint, daß ein Teil der Wirkstoffe sozusagen frei, d. h. nicht eingelagert in eine Matrix, vorliegen kann, so daß dieser Anteil an Wirkstoffen unmittelbar nach der peroralen Applikation freigesetzt wird, während der Anteil an Wirkstoffen, welcher in die Matrix eingelagert ist, kontrolliert bzw. zeitversetzt hierzu freigesetzt wird. Auf diese Weise können Kombinationen unterschiedlicher Wirkstoffe zu unterschiedlichen Zeitpunkten bzw. an unterschiedlichen Resorptionsorten freigesetzt werden, wobei die nicht in eine Matrix eingelagerten Wirkstoffe unmittelbar nach ihrer Applikation bereits im Magen freigesetzt werden, während die in die Matrix eingelagerten Wirkstoffe zeitlich später bzw. versetzt hierzu (z. B. im Dünndarm) freigesetzt werden.

Als Matrixmaterial kann im Rahmen der vorliegenden Erfindung ein geeignetes quellfähiges Polymer verwendet werden. Besonders bewährt haben sich Cellulose und ihre Derivate, vorzugsweise Celluloseether (z. B. Alkylcelluloseether) und Mischungen von verschiedenen Celluloseethern, bevorzugt Hydroxyalkylcellulosen, besonders bevorzugt Hydroxypropylmethylcellulose oder aber eine Mischung von Methylcellulose und gemischten Celluloseethern (Hydroxypropyl-, Hydroxybutylmethylcellulose) (z. B. Methocel^{®}).

Mit derartigen Matrixmaterialien wird erreicht, daß die Wirkstoffe nach ihrer peroralen Applikation derart freigesetzt werden, daß negative Interaktionen zwischen den Wirkstoffen zumindest im wesentlichen vermieden oder aber zumindest minimiert werden können, insbesondere eine negative Beeinflussung der Wirkstoffe in bezug auf ihre Aktivität und/oder ihre Resorption vermieden bzw. minimiert wird.

Vorteilhafterweise setzt die Matrix die Wirkstoffe nach ihrer peroralen Applikation über einen definierten Zeitraum in kontinuierlicher Weise und/oder in jeweils kontrollierten Mengen frei, wodurch die Resorption der Wirkstoffe im Magen-/Darmtrakt optimiert wird, insbesondere eine kurzfristige Überdosierung und/oder Überlastung der Resorptionssysteme zumindest im wesentlichen vermieden wird.

Üblicherweise enthält die erfindungsgemäße Zusammensetzung neben den Wirkstoffen und dem Matrixmaterial außerdem mindestens einen pharmazeutisch unbedenklichen Träger oder Exzipienten.

Neben den vorgenannten Wirkstoffen aus der Gruppe von Vitaminen, Mineralstoffen und/oder Spurenelementen kann die erfindungsgemäße Zusammensetzung darüber hinaus auch noch weitere Wirk- und/oder Inhaltsstoffe enthalten. Solche weiteren Wirk- und/oder Inhaltsstoffe können beispielsweise ausgewählt sein aus der Gruppe von Proteinen und Proteinkonzentraten; Aminosäuren; Glucosaminsalzen und Chondroitinsalzen, insbesondere Sulfaten; Pilzen und Pilzextrakten; Hefen und Hefeextrakten; Algen und Algenextrakten; Kombucha und Kombuchaextraken; Enzymen und Coenzymen; sekundären Pflanzeninhaltsstoffen und den dazugehörigen Pflanzen und Pflanzenextrakten; probiotischen und präbiotischen Kulturen; Fruchtsäuren und deren Salzen; sowie Mischungen der vorgenannten Stoffe und Verbindungen.

Des weiteren kann die erfindungsgemäße Zusammensetzung außerdem mindestens einen weiteren Inhaltsstoff enthalten, beispielsweise Zusatz- und/oder Hilfsstoffe, wie Füll-, Streck-, Binde-, Netz-, Stabilisierungs-, Färbe-, Puffer-, Riech-, Geschmacks-, Süßungs-, Aroma-, Verarbeitungshilfs- und Konservierungsstoffe und -mittel sowie deren Mischungen.

Gemäß einer besonderen Ausführungsform betrifft die vorliegende Erfindung eine Zusammensetzung für die perorale Applikation mit gesteuerter Freisetzung von Wirkstoffen, welche eine Vielzahl bzw. eine Kombination von Wirkstoffen enthält, welche insbesondere ausgewählt sind aus der Gruppe von Vitaminen, Mineralstoffen und Spurenelementen sowie deren Mischungen, wobei die Wirkstoffe in unterschiedlichen Freisetzungssystemen enthalten sind, wobei die Zusammensetzung mindestens zwei, vorzugsweise mindestens drei verschiedene Freisetzungssysteme aufweist, umfassend:
(A) ein erstes Freisetzungssystem mit einem ersten Anteil von Wirkstoffen, insbesondere aus der Gruppe von Vitaminen, Mineralstoffen und/oder Spurenelementen, welches die Wirkstoffe ohne ein Matrixmaterial umfaßt;
(B) ein zweites Freisetzungssystem mit einem zweiten Anteil von Wirkstoffen, insbesondere aus der Gruppe von Vitaminen, Mineralstoffen und/oder Spurenelementen, welches die Wirkstoffe inkorporiert in einer quellfähigen Matrix umfaßt; und
(C) gegebenenfalls ein drittes Freisetzungssystem mit einem dritten Anteil von Wirkstoffen, insbesondere aus der Gruppe von Vitaminen, Mineralstoffen und/oder Spurenelementen, welches die Wirkstoffe inkorporiert in einer quellfähigen Matrix umfaßt;
wobei die Freisetzungssysteme (A), (B) und gegebenenfalls (C) die Wirkstoffe nach peroraler Applikation jeweils kontrolliert und/oder zeitversetzt zueinander freisetzen.

Mit anderen Worten werden gemäß dieser besonderen Ausführungsform die unterschiedlichen Wirkstoffe auf die vorhandenen zwei bzw. drei Freisetzungssysteme (A), (B) und gegebenenfalls (C) verteilt, so daß sie unterschiedlichen Freisetzungsmechanismen unterliegen. Das Freisetzungssystem (A), welches kein Matrixmaterial umfaßt, setzt die Wirkstoffe sehr schnell, insbesondere unmittelbar nach peroraler Applikation, im allgemeinen noch im Magen frei, während die Freisetzungssysteme (B) und (C) aufgrund des Vorhandenseins einer Matrix die Wirkstoffe zu einem späteren Zeitpunkt als das Freisetzungssystem (A) freisetzen und sozusagen eine Semidepot- bzw. Depotwirkung entfalten.

Die verschiedenen Freisetzungssysteme (A), (B) und (C) liegen dabei insbesondere als jeweils diskrete Partikel, insbesondere Formkörper, vorzugsweise Pellets, Agglomerate, Granulate oder dergleichen, vor. Dabei können die diskreten Partikel Teilchendurchmesser im Bereich von 10 bis 1.000 µm, insbesondere 200 bis 750 µm, vorzugsweise 250 bis 600 µm, besonders bevorzugt 300 bis 550 µm, ganz besonders bevorzugt von etwa 500 µm, aufweisen.

Gemäß einer besonderen Ausführungsform können die vorgenannten diskreten Partikel, insbesondere Formkörper, der verschiedenen Freisetzungssysteme (A), (B) und (C) wiederum jeweils zu Preßlingen, insbesondere Tablettenpreßlingen (sogenannten "Minitabletten"), zusammengeführt bzw. verpreßt sein. Diese Preßlinge weisen im allgemeinen Durchmesser von 1.000 bis 3.000 µm, insbesondere 1.200 bis 2.800 µm, vorzugsweise 1.500 bis 2.500 µm, auf. Auf diese Weise werden die einzelnen Freisetzungsphasen verbessert zeitlich voneinander beabstandet und die gewünschte Depotwirkung weiter gesteigert.

Vorteilhafterweise sind die verschiedenen Freisetzungssysteme (A), (B) und (C) zu einer Einheit bzw. Entität zusammengeführt. Hierbei kann es sich insbesondere um eine Tablette (z. B. eine Filmtablette), eine Kapsel, eine Pille, ein Dragee oder dergleichen handeln. Beispielsweise können die einzelnen Partikel der Freisetzungssysteme (A), (B) und (C) zu Preßlingen bzw. "Minitabletten" verpreßt werden, wobei die diskrete Partikelgestalt der einzelnen Freisetzungssysteme und somit ihre Freisetzungscharakteristik in der resultierenden verpreßten Tablette zumindest im wesentlichen erhalten bleibt; die Preßlinge bzw. "Minitabletten" können dann nachfolgend zu einer Gesamttablette (Größe z. B. 10 bis 30 mm, insbesondere 10 bis 25 mm) zusammengeführt bzw. verpreßt sein, welche dann nachfolgend zu einer Filmtablette weiterverarbeitet werden kann, insbesondere durch Überziehen mit geeigneten Polymeren, Lacken etc. (was dem Fachmann als solches bekannt ist).

Im allgemeinen sind die Freisetzungssysteme (A), (B) und (C) derart ausgestaltet, daß sie zumindest teilweise voneinander verschiedene Wirkstoffe enthalten, welche zeitlich versetzt zueinander, insbesondere an unterschiedlichen Resorptionsorten (Magen, Duodenum, Ileum etc.), freigesetzt werden.

Gemäß einer besonderen Ausführungsform können die Freisetzungssysteme (B) und (C) unterschiedliche Mengen an Matrixmaterial enthalten, so daß die in den Freisetzungssystemen (B) und (C) enthaltenen Wirkstoffe zeitversetzt zueinander freigesetzt werden. Die Steuerung der Freisetzung bzw. des Zeitpunkts der Freisetzung erfolgt somit bei dieser Ausführungsform über die Menge desselben Matrixmaterials. Als Matrixmaterialien können die vorgenannten Matrixmaterialien zum Einsatz kommen, d. h. quellfähige Polymere, insbesondere Cellulose oder Cellulosederivate, vorzugsweise Celluloseether und Mischungen von verschiedenen Celluloseethern, bevorzugt Hydroxyalkylcellulosen, besonders bevorzugt Hydroxypropylmethylcellulose oder eine Mischung von Methylcellulose und gemischten Celluloseethern (Hydroxypropyl-, Hydroxybutylmethylcellulose) (z. B. Methocel^{®}).

Bei dieser Ausführungsform, gemäß welcher die Freisetzungssysteme (B) und (C) unterschiedliche Mengen desselben Matrixmaterials enthalten, beträgt die Menge an Matrixmaterial im Freisetzungssystem (B), bezogen auf das Freisetzungssystem (B), im allgemeinen 1 bis 15 Gew.-%, insbesondere 1 bis 10 Gew.-%, vorzugsweise 1 bis 5 Gew.-%, bevorzugt 3 bis 5 Gew.-%, besonders bevorzugt etwa 4 Gew.-%, und beträgt die Menge an Matrixmaterial im Freisetzungssystem (C), bezogen auf das Freisetzungssystem (C), 1 bis 40, insbesondere 2 bis 35 Gew.-%, vorzugsweise 4 bis 10 Gew.-%, bevorzugt 6 bis 8 Gew.-%, besonders bevorzugt etwa 7 Gew.-%. Die vorgenannten Mengenangaben beziehen sich jeweils auf das Trockengewicht.

Das Verhältnis der Menge an Matrixmaterial im Freisetzungssystem (C) zu der Menge an Matrixmaterial im Freisetzungssystem (B) kann dabei im Bereich von 15 : 1 bis 1,1 : 1, insbesondere 10 : 1 bis 1,2 : 1, vorzugsweise 5 : 1 bis 1,5 : 1, besonders bevorzugt 3 : 1 bis 1,5 : 1, variieren. Vorteilhafterweise ist dabei das Verhältnis der Menge an Matrixmaterial im Freisetzungssystem (C) zu der Menge an Matrixmaterial im Freisetzungssystem (B) größer als 1,2 : 1, insbesondere größer als 1,5 : 1, vorzugsweise größer 3 : 1, besonders bevorzugt größer 5 : 1, ist.

Gemäß einer alternativen, erfindungsgemäß aber weniger bevorzugten Ausführungsform können die Freisetzungssysteme (B) und (C) aber auch unterschiedliche Matrixmaterialien erhalten, d. h. die zeitversetzte Freisetzung in den Freisetzungssystemen (B) und (C) kann alternativ auch dadurch erreicht werden, daß unterschiedliche Matrixmaterialien in den Freisetzungssystemen (B) und (C) zum Einsatz kommen, welche nach peroraler Applikation eine unterschiedliche Freisetzungskinetik bewirken und die Wirkstoffe kontrolliert und zeitversetzt zueinander freisetzen.

Gemäß einer besonderen Ausgestaltung kann die erfindungsgemäße Zusammensetzung derart ausgestaltet sein,
- daß das erste Freisetzungssystem (A) als Wirkstoffe Fluoride, insbesondere Natriumfluorid; Kupfersalze, insbesondere Kupfer(II)sulfat; Zinksalze, insbesondere Zinkoxid; Biotin; Vitamine der D-Gruppe, insbesondere Vitamin D₃; Vitamin K₁, Vitamin E oder dessen Derivate, insbesondere Tocopherolacetat; Q 10; Vitamin A; Luteinester; Zeaxanthin; Nicotinamid; Cyanocobalamin umfaßt; und/oder
- daß das zweite Freisetzungssystem (B) als Wirkstoffe Ascorbinsäure; Riboflavin; Folsäure; Thiaminnitrat; Pyridoxinhydrochlorid; Kaliumiodat; Natriummolybdat; Eisensalze, insbesondere Eisen(II)fumarat; Mangansalze, insbesondere Mangan(II)sulfat; Kaliumchlorid; Magnesiumsalze, insbesondere Magnesiumoxid umfaßt; und/oder
- daß das dritte Freisetzungssystem (C) als Wirkstoffe Selensalze, insbesondere Natriumselenit; Calciumpantothenat; Chromsalze, insbesondere Chromchlorid; Calciumhydrogenphosphat umfaßt.

Neben den vorgenannten Wirkstoffen aus der Gruppe von Vitaminen, Mineralstoffen und Spurenelementen können die Freisetzungssysteme (A), (B) und/oder (C) außerdem mindestens einen pharmazeutisch unbedenklichen Träger oder Exzipienten enthalten.

Darüber hinaus können die Freisetzungssysteme (A), (B) und/oder (C) außerdem mindestens einen weiteren Wirk- und/oder Inhaltsstoff enthalten. Ein solcher Wirk- und/oder Inhaltsstoff kann insbesondere ausgewählt sein aus der Gruppe von Proteinen und Proteinkonzentraten; Aminosäuren; Glucosaminsalzen und Chondroitinsalzen, insbesondere Sulfaten; Pilzen und Pilzextrakten; Hefen und Hefeextrakten; Algen und Algenextrakten; Kombucha und Kombuchaextrakten; Enzymen und Coenzymen; sekundären Pflanzeninhaltsstoffen und den dazugehörigen Pflanzen und Pflanzenextrakten; probiotischen und präbiotischen Kulturen; Fruchtsäuren und deren Salzen; sowie deren Mischungen.

Weiterhin können die Freisetzungssysteme (A), (B) und/oder (C) außerdem mindestens einen weiteren Inhaltsstoff enthalten, welcher aus der Gruppe von Zusatz- und/oder Hilfsstoffen ausgewählt ist, wie Füll-, Streck-, Binde-, Netz-, Stabilisierungs-, Färbe-, Puffer-, Riech-, Geschmacks-, Süßungs-, Aroma-, Verarbeitungshilfs- und Konservierungsstoffen und mitteln sowie deren Mischungen.

Im allgemeinen liegt die erfindungsgemäße Zusammensetzung in fester Form vor, insbesondere als Pulver oder Granulat und besonders bevorzugt als Tablette (z. B. Filmtablette), Kapsel, Pille, Dragee oder dergleichen.

Bei der erfindungsgemäßen Zusammensetzung kann es sich insbesondere um ein Arzneimittel oder eine pharmazeutische Zubereitung, ein Medizinprodukt, ein Nahrungsergänzungsmittel oder ein diätetisches Lebensmittel handeln.

Mit der vorliegenden Erfindung sind eine Reihe von Vorteilen verbunden, wie sie nachfolgend skizziert sind:
Die vorliegende Erfindung gewährleistet eine kontrollierte und/oder zeitversetzte Freisetzung von Wirkstoffen, insbesondere von Vitalstoffen (d. h. Vitamine, Mineralstoffe und/oder Spurenelemente). Auf diese Weise ist eine optimale Versorgung mit den Wirkstoffen möglich. Insbesondere kann durch die kontrollierte bzw. zeitversetzte Freisetzung eine negative Interaktion zwischen unterschiedlichen Wirkstoffen zumindest im wesentlichen vermieden oder aber zumindest minimiert werden. Nach peroraler Applikation werden die Wirkstoffe über einen definierten Zeitraum in kontinuierlicher Weise und/oder in jeweils kontrollierten Mengen freigesetzt. Hierdurch wird die Resorption der Wirkstoffe im Magen-/Darmtrakt optimiert. Insbesondere wird eine kurzfristige Überdosierung oder eine Überlastung der Resorptionssysteme zumindest im wesentlichen vermieden.

Bei der erfindungsgemäßen Züsämmensetzung kann eine Freisetzung der wirksamen Bestandteile, insbesondere Vitamine, Mineralstoffe und/oder Spurenelemente, in drei Gruppen bzw. über drei unterschiedliche Freisetzungssysteme erzielt werden: Eine Gruppe mit schneller Freisetzung, eine Gruppe mit Semidepot-Freisetzung (z. B. über ca. zwei Stunden) und eine Depot-Freisetzung (später einsetzend als die Semidepot-Freisetzung und dann über ca. fünf Stunden anhaltend). Diese im Fall der Semidepot und Depot-Freisetzung gewollte, verzögerte Freisetzung erstreckt sich trotz unterschiedlicher Stoffeigenschaften durch die gewählte Verzögerungstechnik auf alle in den jeweiligen Gruppen enthaltenen Wirkstoffe (d. h. Vitamine, Mineralstoffe und/oder Spurenelemente). Dagegen wird für ein orales Arznei- oder Lebensmittel ohne verzögerte Freisetzung erwartet, daß bereits nach etwa zwanzig Minuten in künstlichem Magensaft mindestens 70 % der Wirkstoffe in das umgebende Medium freigesetzt sind.

Die Retardierungen werden durch unterschiedlichen Zusatz bzw. unterschiedliche Mengen von Matrixmaterial, beispielsweise Cellulose oder Cellulosederivate, wie z. B. Hydroxypropylmethylcellulose oder eine Mischung von Methylcellulose und gemischten Celluloseethern (Hydroxypropyl-, Hydroxybutylmethylcellulose) (z. B. Methocel^{®}), erreicht. Das Matrixmaterial quillt im Magen- und Darmsaft und stellt für die enthaltenen Wirksubstanzen eine mechanische und/oder physikochemische Barriere für die Diffusion in die umgebende Magenflüssigkeit oder Darmflüssigkeit dar. Die Semidepot-Freisetzungsgruppe von Wirksubstanzen enthält dabei vorzugsweise weniger Matrixmaterial im Vergleich zur Depot-Freisetzungsgruppe; die erste Freisetzungsgruppe enthält vorzugsweise kein Matrixmaterial. Die drei Freisetzungsgruppen - Freisetzungssysteme (A), (B) und (C) bzw. Freisetzungsgruppen "schnell", "Semidepot" und "Depot" - können dazu jeweils für sich mit allen Wirk- und Inhaltsstoffen granuliert werden und anschließend die einzelnen Granulate homogen gemischt und zu Tabletten verpreßt werden, welche nachfolgend zu Filmtabletten verarbeitet sein können. Jedes Granulat behält in dem entstandenen Tablettenpreßling seine ursprünglichen Eigenschaften hinsichtlich der Retardierung bei. Alternativ ist es gleichermaßen möglich, die verschiedenen Freisetzungsgranulate z. B. in ein Kapselsystem einzubringen.

Die vorliegende Erfindung ermöglicht somit die gezielte bzw. kontrollierte und/oder zeitversetzte Freisetzung von Wirkstoffen aus der Gruppe von Vitaminen, Mineralstoffen und/oder Spurenelemente nach peroraler Applikation, wobei die Vitamine, Mineralstoffe und/oder Spurenelemente gegebenenfalls in Kombination mit weiteren Wirk- bzw. Inhaltsstoffen (z. B. Eiweiße bzw. Eiweißkonzentrate, Aminosäuren, Glucosaminsalze und Chondroitinsalze, insbesondere Sulfate, Pilze und Pilzextrakte, Hefen und Hefeextrakte, Algen und Algenextrakte sowie Kombucha, sekundäre Pflanzeninhaltsstoffe sowie die entsprechenden Pflanzen und Pflanzenextrakte, Coenzyme und Enzyme, probiotische und präbiotische Kulturen, Fruchtsäuren und deren Salze etc.) vorliegen können.

Die erfindungsgemäße Zusammensetzung findet Verwendung insbesondere für Lebensmittel, (ergänzende) bilanzierte Diäten, diätetische Lebensmittel, Medizinprodukte, insbesondere in fester Form (vorzugsweise in Form von Tabletten (z. B. Filmtabletten), Kapseln, Dragees, Pillen, Pulvern und Granulaten und dergleichen).

Die vorliegende Erfindung führt also zu einer insgesamt deutlich vermehrten bzw. verbesserten Aufnahme der angebotenen Wirkstoffe (z. B. Vitamine etc.), und dies über einen gleichermaßen deutlich verlängerten Zeitraum.

Ein weiterer Vorteil der erfindungsgemäßen Zusammensetzung liegt in ihrer relativ einfachen Herstellung. Die Einlagerung der Wirkstoffe in die Matrix erfolgt in an sich bekannter Weise, insbesondere durch Granulierung der in die Matrix einzulagernden Wirkstoffe in Gegenwart der matrixbildenden Materialien.

Außerdem ermöglicht die vorliegende Erfindung ohne weiteres eine große Variabilität in bezug auf die Freisetzungskinetik: Durch Änderung bzw. Variation der Menge und/oder Art des Matrixmaterials läßt sich die Freisetzungskinetik ohne weiteres einstellen bzw. variieren und auf diese Weise sozusagen maßschneidern.

Der Effekt und Nutzen für den Anwender, der mit der erfindungsgemäßen Zusammensetzung erzielt wird, ergibt sich wie folgt:
Die Resorption von Wirkstoffen, insbesondere von Vitaminen, Mineralstoffen und/oder Spurenelementen, erfolgt zu einem großen Teil im Darm. Bei den Resorptionsmechanismen handelt es sich häufig um aktive, d. h. sättigbare Transportsysteme. Zum Teil sind diese Systeme auf wenige Zentimeter Darm beschränkt. Ein schnelles Anfluten der Vitamine bei "normalfreisetzenden" Zubereitungen überlastet schnell die Resorptionssysteme, so daß zum einen nur ein Teil des Vitalstoffangebots aufgenommen werden kann und dies zum anderen nur über einen relativ kurzen Zeitraum erfolgt. Mit der ersten Aufnahme beginnen dann im Körper bereits der Verbrauch und die Ausscheidung. Mit der vorliegenden Erfindung erfolgt - wie zuvor beschrieben - dagegen die Freisetzung gesteuert bzw. kontrolliert. Die freigesetzten Vitalstoffe werden über den ständigen Strom von Mageninhalt in den Darm in relativ geringen, optimalen Konzentrationen zu ihren Resorptionsorten (z. B. Magen, Dünndarm, Dickdarm etc.) transportiert.

Wegen der jeweils relativ geringen Konzentrationen von Vitalstoffen an den einzelnen Wirkorten werden die Resorptionssysteme nicht überlastet, so daß insgesamt über einen längeren Zeitraum (z. B. über einen Zeitraum von fünf Stunden) ein wesentlich größerer Anteil der angebotenen Vitalstoffe resorbiert werden kann. Damit werden auch die Blutspiegel über diesen verlängerten Zeitraum durch ständigen Nachschub aufrechterhalten.

Durch die Eingruppierung der Wirkstoffe bzw. Vitalstoffe in die vorgenannten drei Gruppen - schnelle Freisetzung, Semidepot-Freisetzung und Depot-Freisetzung - werden negative Interaktionen zwischen den Vitaminen, Mineralien und/oder Spurenelementen gemindert bzw. vermieden und damit ebenfalls das Angebot für den Körper erhöht.

Die vorliegende Erfindung führt also zu einer insgesamt deutlich vermehrten und verbesserten Aufnahme der angebotenen Wirkstoffe bzw. Vitalstoffe, und dies über einen ebenfalls deutlich verlängerten Zeitraum.

Auf diese Weise wird die Bioverfügbarkeit der Wirkstoffe bzw. Vitalstoffe signifikant erhöht. Beispielsweise können sich negativ beeinflussende Vitalstoffe (z. B. Kupfer und Zink oder aber Magnesium und Calcium) in unterschiedlichen Phasen freigesetzt werden.

Die Freigabe der Vitalstoffe erfolgt in Abhängigkeit davon, wann mit einer guten Resorption des jeweiligen Vitalstoffs gerechnet werden kann (z. B. im Magen, im Duodenum, im Jejunum, im gesamten Dünndarm, im Dickdarm etc.). So können beispielsweise Glucosamin, Chondroitin, Kupfer und Selen sofort freigesetzt werden, um eine schnelle Verfügbarkeit zu ermöglichen, während andere Vitalstoffe in einer Semidepot- oder Depotphase über Stunden freigesetzt werden können, um eine bedarfsgerechte Langzeitversorgung sicherzustellen.

Weitere Ausgestaltungen, Abwandlungen und Variationen sowie weitere Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung, ohne sie jedoch hierauf zu beschränken.

### Ausführungsbeispiele:

Eine erfindungsgemäße Zusammensetzung für die perorale Applikation mit gesteuerter Freisetzung von Wirkstoffen, welche aus drei Freisetzungssystemen besteht, wird wie nachfolgend beschrieben hergestellt. Während das erste Freisetzungssystem die Wirkstoffe aus der Gruppe von Vitaminen, Mineralstoffen und/oder Spurenelementen ohne ein Matrixmaterial enthält ("schnelle Freisetzungsgruppe"), enthalten die beiden übrigen Freisetzungssysteme die Wirkstoffe aus der Gruppe von Vitaminen, Mineralstoffen und/oder Spurenelementen in ein Matrixmaterial auf der Basis von Hydroxypropylmethylcellulose oder auf der Basis einer Mischung von Methylcellulose und gemischten Celluloseethern (Hydroxypropyl-, Hydroxybutylmethylcellulose) (z. B. Methocel^{®}) inkorporiert, wobei das zweite Freisetzungssystem im Verhältnis zum dritten Freisetzungssystem weniger Matrixmaterial enthält, so daß das zweite Freisetzungssystem als Semidepot-Freisetzungssystem und das dritte Freisetzungssystem als Depot-Freisetzungssystem ausgestaltet ist. Während im zweiten Freisetzungssystem die Menge an Matrixmaterial, bezogen auf das zweite Freisetzungssystem, etwa 4 Gew.-% beträgt, beträgt die Matrixmaterialmenge im dritten Freisetzungssystem, bezogen auf das dritte Freisetzungssystem, etwa 7 Gew.-%.

Zur Herstellung der erfindungsgemäßen Zusammensetzung werden die drei Freisetzungssysteme, jeweils für sich, mit allen Wirk- und Inhaltsstoffen in an sich bekannter Weise granuliert (Granulatgröße: z. B. ca. 500 µm). Die resultierenden Granulate der drei Freisetzungssysteme werden anschließend homogen miteinander vermischt und zu Tabletten ("Minitabletten") verpreßt (Preßlingsgröße: ca. 1.500 bis 2.500 µm), wobei jedes Freisetzungsgranulat in den entstandenen Tablettenpreßlingen seine ursprünglichen Eigenschaften hinsichtlich der kontrollierten Freisetzung bzw. Retardierung beibehält. Die einzelnen Tablettenpreßlinge ("Minitabletten") wiederum können dann zu einer Gesamteinheit (z. B. Kapsel, Dragee, Tablette etc.) zusammengeführt werden, insbesondere durch Verpressen zu einer Tablette (Tablettengröße: ca. 15 bis 20 mm), die vorteilhafterweise gegebenenenfalls mit einem polymeren Fim bzw. Lack überzogen werden kann, um eine Fimtablette zu erhalten.

In der nachstehenden Tabelle 1 sind die in der erfindungsgemäßen Zusammensetzung enthaltenen Vitalstoffe (d. h. Vitamine, Mineralstoffe und Spurenelemente) mit ihrem jeweiligen Resorptionsort, der jeweiligen Resorptionsart und ihrer jeweils vorhandenen Menge wiedergegeben, während in der nachfolgenden Tabelle 2 die Zuordnung bzw. Inkorporierung der einzelnen Bestandteile in die einzelnen Freisetzungsgruppen wiedergegeben ist.

In der nachstehenden Tabelle 3 ist eine alternative erfindungsgemäße Zusammensetzung mit den darin enthaltenen Vitalstoffen angegeben.

**Tabelle 1**

| **Rohstoff** | **UD** | **Resorptionsort** | **Resorptionsart** | **Resorptionsort** | **Mengen ohne UD** | **Menge pro Kern inkl. UD** |
|---|---|---|---|---|---|---|
| Cyanocobalamin 0,1%ig | 20% | Jejunum/Ileum | a | 1 | 1,0000 | 1,2000 |
| Vitamin D3 | 20% | Darm | p | 1 | 2,0000 | 2,4000 |
| Pyridoxinhydrochlorid | 10% | Jejunum/Ileum | p | 2 | 2,4334 | 2,6767 |
| Riboflavin | 35 % | Duodenum | a/p | 2 | 1,6000 | 2,1600 |
| Thiaminnitrat | 10 % | Duodenum | a/p | 2 | 1,7270 | 1,8997 |
| Folsäure | 40% | Darm | a/p | 2 | 0,2000 | 0,2800 |
| Biotin | 20% | Duodenum | a/p | 1 | 0,1500 | 0,1800 |
| Q10 | | | | 1 | | 0,2500 |
| Kaliumiodat | | Proximaler Intest. | a | 2 | | 0,3373 |
| Natriumfluorid | | Magen/Dünndarm | p | 1 | | 1,1051 |
| Vitamin K1 5%ig | 5% | Darm (auch Colon) | p/a | 1 | 1,4 | 1,4700 |
| Chrom(II)chlorid*6H2O | | | p ? | 3 | | 0,1500 |
| Natriummolybdat*2H2O | | Dünndarm | p | 2 | | 0,1260 |
| Natriumselenit*5H2O | | Dünndarm | a/p | 3 | | 0,1000 |
| Zinkoxid | | Duodenum/Jejunum | a/p | 1 | | 6,2200 |
| Kupfer(II)sulfat*5H2O | | Magen/Duodenum | a/p | 1 | | 3,9290 |
| Mangan(II)sulfat*H2O | | Dünndarm | | 2 | | 1,5380 |
| Kaliumchlorid | | Duodenum | a | 2 | | 19,2100 |
| Nicotinamid | 5% | Dünndarm | a/p | 1 | 18,0000 | 18,9000 |
| Calciumpantothenat | 5% | Dünndarm | p | 3 | 6,5460 | 6,8733 |
| Vitamin A (Pulver) | 5% | Darm | p | 1 | 5,0000 | 5,2500 |
| Luteinester (10 %) | | Darm | p | 1 | | 3,0000 |
| Zeaxanthin (Beadlets 25 %) | | Darm | p | 1 | | 2,0000 |
| Calciumhydrogenphospat (Di-Cafos) | | Duodenum/Jejunum | a/p | 3 | | 343,5200 |
| Magnesiumoxid granuliert (6,5 % Glühverlust) | | Ileum | a/p | 2 | | 53,2050 |
| Ascorbinsäure (90 %) | 10% | Duodenum | a/p | 2 | 66,6667 | 73,3334 |
| Tocopherolaceta (505CWS/S) | | Duodenum | p | 1 | 10 mg Vitamin E | 29,8600 |
| Eisen(II)fumarat | | Dünndarm | a | 2 | | 45,7940 |
| Methocel K 100 M | | | | | | 27,5000 |
| Boeson | | | | | | 40,0000 |
| Magnesiumstearat | | | | | | 5,0000 |
| | | | | | | 699,4675 |
| | | Duodenum = oberer Dünndarm!!! | | a=aktiv/passiv | | |
| Allgemeines: | | | | | | |
| Duodenum, Jejunum und in geringem Umfang Ileum sind die Hauptorte der resoptiven Vorgänge. Im Dickdarm überwiegend Resorption; von Wasser und den Elektrolyten (insbesondere Na und Cl), die anderen Nährstoffe werden bakteriell verstoffwechselt. | | | | | | |
| Resorptionsort 1: Magen/Duodenum . | | | | | | |
| Resorptionsort 2: Jejunum | | | | | | |
| Resorptionsort 3: Ileum | | | | | | |

**Tabelle 2**

| **Resorption** | **Bemerkungen** |
|---|---|
| **Gruppe 1 ("schnell")** | |
| Natriumfluorid | |
| Kuper(II)sulfat*5H2O | neg. Interaktion mit Zn nur bei sehr hohen unphysiolog. Cu-Konz. |
| Zinkoxid | hier: keine negativen Interaktionen mit Ca, Fe, Cr (Cu nur bei sehr hohen unphysiolog. Zn-Konz.) |
| Biotin | |
| Vitamin D3 | fettlösliche Vitamine brauchen Fett bzw. Micellen für Resorption daher wichtig, daß schnell freigesetzt und mit Fetten interagieren |
| Vitamin K1 5 %ig | |
| Tocopherolacetat 505CWS/S | |
| Q10 | Q 10 auch verbessert in Anwesenheit von Fett resorbiert |
| Vitamin A Pulver | |
| Luteinester 10 % | Carotinoide wie fettlösliche Vitamine resorbiert |
| Zeaxanthin Beadlets 25 % | Carotinoide wie fettlösliche Vitamine resorbiert |
| Nicotinamid | beginnt schon im Magen |
| Cyanocobalamin 0.1 %ig | B12 braucht intrinsic factor aus Magenschleimhaut für Resorption; Bindung an IF im Duodenum; Gruppe 2 ginge auch |
| | |

| **Gruppe 2 ("semidepot")** | |
|---|---|
| Ascorbinsäure 90 % | verbessert Fe-Resorption signifikant |
| Riboflavin | |
| Folsäure | |
| Thiaminnitrat | |
| Pyridoxinhydrochlorid | im gesamten Dünndarm |
| Kaliumiodat | könnte auch in Gruppe 3 (fast vollständig resorbiert im "GIT") |
| Natriummolybdat*2H2O | könnte auch in Gruppe 3 (zu 80 % im "Dünndarm" resorbiert) |
| Eisen(II)fumarat | |
| Mangan(II)sulfat*H2O | könnte auch in Gruppe 3 (in geringen Mengen im "GIT"), aber Ca könnte verhindern |
| Kaliumchlorid | |
| Magnesiumoxid granuliert (6,5 % Glühverlust) | |
| | |

| **Gruppe 3 ("depot")** | |
|---|---|
| Natriumselenit*5H2O | |
| Calciumpantothenat | |
| Chromchlorid | Cr verträgt sich nicht mit Zn und Fe |
| Calciumhydrogenphosphat (Di-Cafos) | Resorption u. a. in Duodenum und Jeiunum |
| | |
| **Bioflavonoide in allen 3 Phasen (ca. 20 mg)** | |
| Allgemeines: | |
| Duodenum, Jejunum und in geringem Umfang Ileum sind die Hauptorte der resoptiven Vorgänge. Im Dickdarm überwiegend Resorption von Wasser und den Elektrolyten (insbesondere Na und Cl), die anderen Nährstoffe werden bakteriell verstoffwechselt. | |
| Resorptionsort 1: | Magen/Duodenum |
| Resorptionsort 2: | Jejunum |
| Resorptionsort 3: | Ileum |

**Tabelle 3**

| **Rohstoff** | **Menge Mineral/ Vitamin / Tbl.** | **UD** | **Mengen ohne UD** | **Menge pro Kern inkl. UD** | **Prozentuale Zusammensetzung, trocken** |
|---|---|---|---|---|---|
| **Granulat Phase 1 = direkt freisetzend** | | | | | |
| Cyanocobalamin 0,1%ig | 1 µg B12 | 20% | 1,0000 | 1,2000 | 0,5434 |
| Vitamin D3 | 5 µg D3 | 20% | 2,0000 | 2,4000 | 1,0869 |
| Biotin | 150 µg | 20% | 0,1500 | 0,1800 | 0,0815 |
| Q10 | 250 µg | | | 0,2500 | 0,1132 |
| Natriumfluorid | 0,5 mg F | | | 1,1051 | 0,5005 |
| Vitamin K1 5%ig | 70 µg K1 | 5% | 1,4 | 1,4700 | 0,6657 |
| Zinkoxid | 5 mg Zn | | | 6,2200 | 2,8168 |
| Kupfer(II)sulfat*5H2O | 1 mg Cu | | | 3,9290 | 1,7793 |
| Nicotinamid | 18 mg | 5% | 18,0000 | 18,9000 | 8,5591 |
| Vitamin A (Pulver) | 800 µg | 5% | 5,3333 | 5,6000 | 2,5360 |
| Luteinester (5%) | 300 µg Lutein | | | 12,0000 | 5,4343 |
| Tocopherolacetat (50%) CWS/S | 10 mg | | 10 mg Vitamin | 29,8000 | 13,4952 |
| Magnesiumoxid | 45 mg Mg | | | 79,8075 | 36,1416 |
| Zeaxanthin (20%) | 500 µg | | | 2,5000 | 1,1321 |
| Citrus-Bioflavonoide | | | | 6,7000 | 3,0342 |
| CaboSil | | | | 3,7500 | 1,6982 |
| Avicel PH101 | | | | 32,1073 | 14,5401 |
| Vivasol | | | | 4,4000 | 1,9926 |
| Kollidon 25 | | | | 8,5000 | 3,8493 |
| Isopropanol | | | | 0,0000 | 0,0000 |
| | | | **Phase 1 gesamt**: | **220,8189** | **100,0000** |
| | | | | | |

| **Granulat Phase 2 = mittel freisetzend** | | | | | |
|---|---|---|---|---|---|
| Pyridoxinhydrochlorid | 2 mg | 10% | 2,4334 | 2,6767 | 1,4446 |
| Riboflavin | 1,6 mg | 35 % | 1,6000 | 2,1600 | 1,1658 |
| Thiaminnitrat | 1,4 mg | 10% | 1,7270 | 1,8997 | 1,0253 |
| Folsäure | 200 µg | 40% | 0,2 | 0,2800 | 0,1511 |
| Kaliumjodat | 100 µg J | | | 0,1687 | 0,0910 |
| Natriummolybdat*2H2O | 50 µg Mo | | | 0,1260 | 0,0680 |
| Mangan(II)sulfat*H2O | 0,5 mg Mn | | | 1,5380 | 0,8301 |
| Kaliumchlorid | 10 mg K | | | 19,2100 | 10,3677 |
| Ascorbinsäure (90%) ohne Lactose | 60 mg | 10% | 66,6667 | 79,3334 | 39,5782 |
| Eisen(II)fumarat | 14 mg Fe | | | 42,5930 | 22,9875 |
| Bioflavonoide | | | | 6,7000 | 3,6160 |
| Methocel F4 Premium | | | | 19,0000 | 10,2543 |
| CaboSil | | | | 5,5000 | 2,9684 |
| Kollidon 25 | | | | 10,1020 | 5,4521 |
| Isopropanol | | | | 0,0000 | 0,0000 |
| | | | | 185,2875 | 100,0000 |

| **Pressmischung Phase 2** | | | | | |
|---|---|---|---|---|---|
| **Granulat Phase 2** | | | | 185,2875 | 98,9829 |
| Magnesiumstearat | | | | 1,9040 | 1,0171 |
| | | | | **187,1915** | **100,0000** |

| **Granulat Phase 3** = **langsam freisetzend** | | | | | |
|---|---|---|---|---|---|
| Chrom(III)chlorid*6H2O | 30 µg Cr | | | 0,1500 | 0,0182 |
| Natriumselenit*5H2O | 30 µg Se | | | 0,1 | 0,0122 |
| Calciumpantothenat | 6 mg Pantothens. | 5% | 6,546 | 6,8733 | 0,8354 |
| Calciumhydrogenphosphat(Di-Cafos) | 80 mg Ca/60 mg P | | | 343,5200 | 41,7537 |
| Calciumcarbonat | 40 mg Ca | | | 99,9052 | 12,1431 |
| Methocel F4 Premium | | | | 272,8515 | 33,1642 |
| Bioflavonoide | | | | 6,6000 | 0,8022 |
| CaboSit | | | | 20,0000 | 2,4309 |
| Kollidon 25 | | | | 72,7300 | 8,8401 |
| Isopropanol | | | | 0,0000 | 0,0000 |
| | | | | 822,7300 | 100,0000 |

| **Pressmischung Phase 3** | | | | | |
|---|---|---|---|---|---|
| Granulat Phase 3 | | | | 822,7300 | 99,0000 |
| Magnesiumstearat | | | | 8,3100 | 1,0000 |
| | | | | **831,0400** | 100,0000 |

| **Pressmischung Vitalstoffe A-Z** | | | | | |
|---|---|---|---|---|---|
| Granulat Phase 1 | | | | 220,819 | 14,8300 |
| Minikerne Phase 2 | | | | 187,192 | 12,5716 |
| Minikerne Phase 3 | | | | 831,040 | 55,8119 |
| Cellulosepulver | | | | 79,600 | 5,3459 |
| Boeson VP | | | | 79,600 | 5,3459 |
| Avicel PH101 | | | | 36,560 | 2,4553 |
| Vivasol | | | | 43,000 | 2,8878 |
| Cab-O-Sil | | | | 7,450 | 0,5003 |
| Magnesiumstearat | | | | 3,740 | 0,2512 |
| | | | Kern gesamt: | **1489,000** | 100,0000 |
| | | | | | |
| | | | | | |

| **Filmtablette** | | | **UD** | | |
|---|---|---|---|---|---|
| Kern | 1489,000 | 1489,000 | 0% | 1489,0004 | |
| Lecithin | 15 | 15,000 | 10% | 16,5000 | |
| Sepifilm LP10 | 21 | 21,000 | 10% | 23,1000 | |
| Talkum | 9 | 9,000 | 10% | 9,9000 | |
| Wasser | 0 | 270 | 10% | 297,0000 | |
| Coating gelb Opadry II 45U32252 | 46 | 46 | 10% | 50,6000 | |
| Wasser | 0 | 307,85 | 10% | 338,6350 | |
| | 1580,000 | 2157,850 | | 2224,7354 | |

## Patentansprüche

1. Zusammensetzung für die perorale Applikation mit gesteuerter Freisetzung von Wirkstoffen, enthaltend eine Vielzahl und/oder eine Kombination von Wirkstoffen, ausgewählt aus der Gruppe von Vitaminen, Mineralstoffen und Spurenelementen sowie deren Mischungen, wobei die Wirkstoffe in unterschiedlichen Freisetzungssystemen enthalten sind, wobei die Zusammensetzung mindestens drei verschiedene Freisetzungssysteme aufweist, umfassend:
(A) ein erstes Freisetzungssystem mit einem ersten Anteil von Wirkstoffen aus der Gruppe von Vitaminen, Mineralstoffen und/oder Spurenelementen, welches die Wirkstoffe ohne ein Matrixmaterial umfaßt;
(B) ein zweites Freisetzungssystem mit einem zweiten Anteil von Wirkstoffen aus der Gruppe von Vitaminen, Mineralstoffen und/oder Spurenelementen, welches die Wirkstoffe inkorporiert in einer quellfähigen Matrix umfaßt; und
(C) ein drittes Freisetzungssystem mit einem dritten Anteil von Wirkstoffen aus der Gruppe von Vitaminen, Mineralstoffen und/oder Spurenelementen, welches die Wirkstoffe inkorporiert in einer quellfähigen Matrix umfaßt,
- wobei die Freisetzungssysteme (A), (B) und (C) die Wirkstoffe nach peroraler Applikation jeweils kontrolliert und zeitversetzt zueinander freisetzen,
- wobei die Freisetzungssysteme (A), (B) und (C) jeweils diskrete Partikel mit Teilchendurchmessern im Bereich von 10 bis 1.000 µm umfassen,
- wobei die Freisetzungssysteme (A), (B) und (C) zu einer Einheit zusammengeführt sind,
- wobei die Freisetzungssysteme (A), (B) und (C) zumindest teilweise voneinander verschiedene Wirkstoffe enthalten und
- wobei die Freisetzungssysteme (B) und (C) unterschiedliche Mengen an Matrixmaterial enthalten, so daß die in den Freisetzungssystemen (B) und (C) enthaltenen Wirkstoffe zeitversetzt zueinander freigesetzt werden, wobei das Verhältnis der Menge an Matrixmaterial im Freisetzungssystem (C) zu der Menge an Matrixmaterial im Freisetzungssystem (B) im Bereich von 15 : 1 bis 1,1 : 1 variiert.

2. Zusammensetzung nach Anspruch 1, **dadurch gekennzeichnet, daß** die Matrix bei Kontakt mit wäßrigen fluiden Medien, insbesondere bei Kontakt mit Magenund/oder Darmsaft, quellfähig ist.

3. Zusammensetzung nach Anspruch 1 oder 2, **dadurch gekennzeichnet, daß** die Matrix ein quellfähiges Polymer, insbesondere Cellulose oder ein Cellulosederivat, vorzugsweise einen Celluloseether oder eine Mischung von verschiedenen Celluloseethern, bevorzugt eine Hydroxyalkylcellulose, besonders bevorzugt Hydroxypropylmethylcellulose oder aber eine Mischung von Methylcellulose und gemischten Celluloseethern, wie Hydroxypropyl-Hydroxybutylmethylcellulose, umfaßt oder hieraus besteht.

4. Zusammensetzung nach einem oder mehreren der vorangehenden Ansprüche, **dadurch gekennzeichnet, daß** die Matrix die Wirkstoffe nach peroraler Applikation derart freisetzt, daß negative Interaktionen zwischen den Wirkstoffen zumindest im wesentlichen vermieden oder aber zumindest minimiert werden, und/oder daß die Matrix die Wirkstoffe nach peroraler Applikation über einen definierten Zeitraum in kontinuierlicher Weise und/oder in jeweils kontrollierten Mengen freisetzt, wodurch die Resorption der Wirkstoffe im Magen-/Darmtrakt optimiert wird, insbesondere eine kurzfristige Überdosierung und/oder Überlastung der Resorptionssysteme zumindest im wesentlichen vermieden wird.

5. Zusammensetzung nach einem oder mehreren der vorangehenden Ansprüche, **dadurch gekennzeichnet, daß** die diskreten Partikel Formkörper, vorzugsweise Pellets, Agglomerate, Granulate oder dergleichen, sind.

6. Zusammensetzung nach einem oder mehreren der vorangehenden Ansprüche, **dadurch gekennzeichnet, daß** die diskreten Partikel Teilchendurchmesser im Bereich von 200 bis 750 µm, vorzugsweise 250 bis 600 µm, besonders bevorzugt 300 bis 550 µm, ganz besonders bevorzugt von etwa 500 µm, aufweisen.

7. Zusammensetzung nach einem oder mehreren der vorangehenden Ansprüche, **dadurch gekennzeichnet, daß** die diskreten Partikel, insbesondere Formkörper, der verschiedenen Freisetzungssysteme (A), (B) und (C) jeweils zu Preßlingen, insbesondere Tablettenpreßlingen ("Minitabletten"), zusammengeführt bzw. verpreßt sind, insbesondere wobei die Preßlinge Durchmesser von 1.000 bis 3.000 µm, insbesondere 1.200 bis 2.800 µm, vorzugsweise 1.500 bis 2.500 µm, aufweisen.

8. Zusammensetzung nach einem oder mehreren der vorangehenden Ansprüche, **dadurch gekennzeichnet, daß** die verschiedenen Freisetzungssysteme (A), (B) und (C) zu einer Einheit in Form einer Tablette, insbesondere Filmtablette, einer Kapsel, einer Pille oder eines Dragees zusammengeführt sind.

9. Zusammensetzung nach Anspruch 7 oder 8, **dadurch gekennzeichnet, daß** die Preßlinge, insbesondere Tablettenpreßlinge ("Minitabletten"), zu einer Gesamttablette zusammengeführt bzw. verpreßt sind, insbesondere wobei die Gesamttablette Durchmesser von 10 bis 30 mm, insbesondere 10 bis 25 mm, aufweist und/oder insbesondere wobei die Gesamttablette zu einer Filmtablette verarbeitet ist.

10. Zusammensetzung nach einem oder mehreren der vorangehenden Ansprüche, **dadurch gekennzeichnet, daß** die Menge an Matrixmaterial im Freisetzungssystem (B), bezogen auf das Freisetzungssystem (B), 1 bis 15 Gew.-%, insbesondere 1 bis 10 Gew.-%, vorzugsweise 1 bis 5 Gew.-%, bevorzugt 3 bis 5 Gew.-%, beträgt und/oder daß die Menge an Matrixmaterial im Freisetzungssystem (C), bezogen auf das Freisetzungssystem (C), 1 bis 40, insbesondere 2 bis 35 Gew.-%, vorzugsweise 4 bis 10 Gew.-%, bevorzugt 6 bis 8 Gew.-%, beträgt.

11. Zusammensetzung nach einem oder mehreren der vorangehenden Ansprüche, **dadurch gekennzeichnet, daß** das Verhältnis der Menge an Matrixmaterial im Freisetzungssystem (C) zu der Menge an Matrixmaterial im Freisetzungssystem (B) im Bereich von 10 : 1 bis 1,2 : 1, vorzugsweise 5 : 1 bis 1,5 : 1, besonders bevorzugt 3 : 1 bis 1,5 : 1, variiert und/oder daß das Verhältnis der Menge an Matrixmaterial im Freisetzungssystem (C) zu der Menge an Matrixmaterial im Freisetzungssystem (B) größer als 1,2 : 1, insbesondere größer als 1,5 : 1, vorzugsweise größer 3 : 1, besonders bevorzugt größer 5 : 1, ist.

12. Zusammensetzung nach einem oder mehreren der vorangehenden Ansprüche, **dadurch gekennzeichnet, daß** die Freisetzungssysteme (B) und (C) unterschiedliche Matrixmaterialien enthalten, so daß die in den Freisetzungssystemen (B) und (C) enthaltenen Wirkstoffe zeitversetzt zueinander freigesetzt werden.

13. Zusammensetzung nach einem oder mehreren der vorangehenden Ansprüche, **dadurch gekennzeichnet,**
**daß** das erste Freisetzungssystem (A) als Wirkstoffe Fluoride, insbesondere Natriumfluorid; Kupfersalze, insbesondere Kupfer(II)sulfat; Zinksalze, insbesondere Zinkoxid; Biotin; Vitamine der D-Gruppe, insbesondere Vitamin D₃; Vitamin K₁, Vitamin E oder dessen Derivate, insbesondere Tocopherolacetat; Q 10; Vitamin A; Luteinester; Zeaxanthin; Nicotinamid; Cyanocobalamin umfaßt; und/oder
**daß** das zweite Freisetzungssystem (B) als Wirkstoffe Ascorbinsäure; Riboflavin; Folsäure; Thiaminnitrat; Pyridoxinhydrochlorid; Kaliumiodat; Natriummolybdat; Eisensalze, insbesondere Eisen(II)fumarat; Mangansalze, insbesondere Mangan(II)sulfat; Kaliumchlorid; Magnesiumsalze, insbesondere Magnesiumoxid umfaßt; und/oder
**daß** das dritte Freisetzungssystem (C) als Wirkstoffe Selensalze, insbesondere Natriumselenit; Calciumpantothenat; Chromsalze, insbesondere Chromchlorid; Calciumhydrogenphosphat umfaßt.

14. Zusammensetzung nach einem oder mehreren der vorangehenden Ansprüche, **dadurch gekennzeichnet, daß** die Freisetzungssysteme (A), (B) und/oder (C) au-βerdem mindestens einen pharmazeutisch unbedenklichen Träger oder Exzipienten enthalten.

15. Zusammensetzung nach einem oder mehreren der vorangehenden Ansprüche, **dadurch gekennzeichnet, daß** die Freisetzungssysteme (A), (B) und/oder (C) au-βerdem mindestens einen weiteren Wirk- und/oder Inhaltsstoff enthalten, ausgewählt aus der Gruppe von Proteinen und Proteinkonzentraten; Aminosäuren; Glucosaminsalzen und Chondroitinsalzen, insbesondere Sulfaten; Pilzen und Pilzextrakten; Hefen und Hefeextrakten; Algen und Algenextrakten; Kombucha und Kombuchaextrakten; Enzymen und Coenzymen; sekundären Pflanzeninhaltsstoffen und den dazugehörigen Pflanzen und Pflanzenextrakten; probiotischen und präbiotischen Kulturen; Fruchtsäuren und deren Salzen; sowie deren Mischungen.

16. Zusammensetzung nach einem oder mehreren der vorangehenden Ansprüche, **dadurch gekennzeichnet, daß** die Freisetzungssysteme (A), (B) und/oder (C) au-βerdem mindestens einen weiteren Inhaltsstoff enthalten, insbesondere ausgewählt aus der Gruppe von Zusatz- und/oder Hilfsstoffen, wie Füll-, Streck-, Binde-, Netz-, Stabilisierungs-, Färbe-, Puffer-, Riech-, Geschmacks-, Süßungs-, Aroma-, Verarbeitungshilfs- und Konservierungsstoffen und -mitteln sowie deren Mischungen.

17. Zusammensetzung nach einem oder mehreren der vorangehenden Ansprüche, **dadurch gekennzeichnet, daß** die Zusammensetzung in fester Form vorliegt, insbesondere als Pulver oder Granulat oder vorzugsweise als Tablette, insbesondere Filmtablette, Kapsel, Pille oder Dragee.

18. Zusammensetzung nach einem oder mehreren der vorangehenden Ansprüche, **dadurch gekennzeichnet, daß** die Zusammensetzung ein Arzneimittel oder eine pharmazeutische Zubereitung, ein Medizinprodukt, ein Nahrungsergänzungsmittel oder ein diätetisches Lebensmittel ist.

## Claims

1. A composition for peroral application, having controlled release of active ingredients, containing a plurality and/or a combination of active ingredients, selected from the group of vitamins, minerals, and trace elements, as well as the mixtures thereof, wherein the active ingredients are comprised in different release systems, wherein the composition has at least three different release systems, comprising:
(A) a first release system having a first proportion of active ingredients from the group of vitamins, minerals, and/or trace elements comprising the active ingredients without any matrix material;
(B) a second release system having a second proportion of active ingredients from the group of vitamins, minerals, and/or trace elements, incorporating the active ingredients in a swellable matrix; and
(C) a third release system having a third proportion of active ingredients from the group of vitamins, minerals, and/or trace elements, incorporating the active ingredients in a swellable matrix,
- wherein the release systems (A), (B), and (C) each release the active ingredients after peroral application in a controlled and time-delayed manner,
- wherein the release systems (A), (B), and (C) each comprise discrete particles having particle diameters within the range of 10 to 1,000 µm,
- wherein the release systems (A), (B), and (C) are each combined into one unit,
- wherein the release systems (A), (B), and (C) contain active ingredients that are at least partially different from each other, and
- wherein the release systems (B) and (C) contain different amounts of matrix material such that the active ingredients contained in the release systems (B) and (C) are released in a time-delayed manner of each other, wherein the ratio of the amount of matrix material in the release system (C) to the amount of matrix material in the release system (B) varies within a range of 15:1 to 1.1:1.

2. The composition according to claim 1, **characterized in that** the matrix is swellable upon contact with aqueous, fluid media, in particular with the contact with gastric juice and/or chyle.

3. The composition according to claims 1 or 2, **characterized in that** the matrix comprises or consists of a swellable polymer, in particular cellulose or a cellulose derivative, preferably cellulose ether, or a mixture of different cellulose ethers, preferably hydroxy alkyl cellulose, particularly preferred hydroxy propyl methyl cellulose, or a mixture of methyl cellulose and mixed cellulose ethers, such as hydroxy propyl hydroxy butyl methyl cellulose.

4. The composition according to one or more of the previous claims, **characterized in that** the matrix releases the active ingredients after peroral application such that negative interactions between the active ingredients are at least essentially avoided, or at least lessened, and/or that the matrix releases the active ingredients after peroral application over a defined time period in a continuous manner, and/or each in controlled amounts, by means of which the resorption of the active ingredients are optimized within the gastrointestinal tract, in particular a short-term overdosage, and/or an overload of the resorption systems is at least essentially avoided.

5. The composition according to one or more of the previous claims, **characterized in that** the discrete particles are molds, preferably pellets, agglomerates, granulates, or the like.

6. The composition according to one or more of the previous claims, **characterized in that** the discrete particles have particle diameters within the range of 200 to 750 µm, preferably 250 to 600 µm, particularly preferred 300 to 550 µm, especially preferred about 500 µm.

7. The composition according to one or more of the previous claims, **characterized in that** the discrete particles, in particular the molds, of the various release systems (A), (B), and (C) are each consolidated or pressed into pellets, in particular tablet pellets ("mini-tablets"), in particular wherein the pellets have diameters of 1,000 to 3,000 µm, in particular 1,200 to 2,800 µm, preferably 1,500 to 2,500 µm.

8. The composition according to one or more of the previous claims, **characterized in that** the various release systems (A), (B), and (C) are consolidated into one unit in the form of a tablet, in particular a coated tablet, a capsule, a pill, or coated pills.

9. The composition according to claims 7 or 8, **characterized in that** the pellets, in particular the tablet pellets ("mini-tablets"), are consolidated or pressed into a total tablet, in particular wherein the total tablet has a diameter of 10 to 30 mm, in particular 10 to 25 mm, and/or in particular, wherein the total tablet is processed into a coated tablet.

10. The composition according to one or more of the previous claims, **characterized in that** the amount of matrix material in the release system (B), based on the release system (B), is 1 to 15 % by weight, in particular 1 to 10 % by weight, preferably 1 to 5 % by weight, more preferably 3 to 5 % by weight, and/or that the amount of matrix material in the release system (C), based on the release system (C), is 1 to 40, in particular 2 to 35 % by weight, preferably 4 to 10 % by weight, more preferably 6 to 8 % by weight.

11. The composition according to one or more of the previous claims, **characterized in that** the ratio of the amount of matrix material in the release system (C) to the amount of matrix material in the release system (B) varies within the range of 10:1 to 1.2:1, preferably 5:1 to 1.5:1, particularly preferred 3:1 to 1.5:1, and/or that the ratio of the amount of matrix material in the release system (C) to the amount of matrix material in the release system (B) is greater than 1.2:1, in particular greater than 1.5:1, preferably greater than 3:1, more preferably greater than 5:1.

12. The composition according to one or more of the previous claims, **characterized in that** the release systems (B) and (C) contain different matrix materials such that the active ingredients contained in the release systems (B) and (C) are released at a time delay of each other.

13. The composition according to one or more of the previous claims, **characterized in that**
the first release system (A) comprises fluorides, in particular sodium fluoride; copper salt, in particular copper(II)sulfate; zinc salts, in particular zinc oxide; biotin; vitamins of the group D, in particular vitamin D₃; vitamin K₁, vitamin E, or the derivatives thereof, in particular tocopherol acetate; Q 10; vitamin A; lutein ester; zeaxanthin; nicotine amide; cyanocobalamine as the active ingredients; and/or
the second release system (B) comprises ascorbic acid; riboflavin; folic acid, thiamin nitrate; pyriodoxin hydrochloride; potassium iodate; sodium molybdate; iron salts, in particular iron(II)fumarate; manganese salts, in particular manganese(II)sulfate; potassium chloride; magnesium salts, in particular magnesium oxide as the active ingredients; and/or
the third release system (C) comprises selenium salts, in particular sodium selenite; calcium panthothenate; chromate salts, in particular chromate chloride; calcium hydrogen phosphate as the active ingredients.

14. The composition according to one or more of the previous claims, **characterized in that** the release systems (A), (B), and/or (C) further contain at least one pharmaceutically acceptable carrier or excipient.

15. The composition according to one or more of the previous claims, **characterized in that** the release systems (A), (B), and/or (C) further contain at least one additional active ingredient and/or substance, selected from the group of proteins and protein concentrates; amino acids; glucosamine salts and chondroitin salts, in particular sulfates; fungi and fungus extracts; yeasts and yeast extracts; algae and algae extracts; kombucha and kombucha extracts; enzymes and coenyzmes; secondary plant ingredients and the related plants and plant extracts; probiotic and prebiotic cultures; fruit acids and the salts thereof; as well as the mixtures thereof.

16. The composition according to one or more of the previous claims, **characterized in that** the release systems (A), (B), and/or (C) further contain at least one additional substance, in particular selected from the group of additives and/or excipients, such as fillers, extenders, binders, wetting agents, stabilizers, colorants, buffers, smelling, tasting, sweetening, aromatic agents, processing aids, and preservatives and preserving agents, as well as the mixtures thereof.

17. The composition according to one or more of the previous claims, **characterized in that** the composition is present as a solid, in particular as a powder or granulate, or preferably as a tablet, in particular as a coated tablet, capsule, pill, or coated pill.

18. The composition according to one or more of the previous claims, **characterized in that** the composition is a pharmaceutical agent, or a pharmaceutical preparation, a medical product, a dietary supplement, or a dietary food.

## Revendications

1. Composition pour application perorale à libération contrôlée d'agents actifs, contenant une pluralité et/ou une combinaison d'agents actifs, choisis dans le groupe constitué par les vitamines, les minéraux et les oligoéléments, ainsi que leurs mélanges, les agents actifs étant contenus dans différents systèmes de libération, la composition comprenant au moins trois systèmes de libération différents, comprenant:
(A) un premier système de libération contenant une première proportion d'agents actifs du groupe constitué par les vitamines, les minéraux et/ou les oligoéléments, qui comprend les agents actifs sans matériau de matrice;
(B) un deuxième système de libération contenant une deuxième proportion d'agents actifs du groupe constitué par les vitamines, les minéraux et/ou les oligoéléments, qui comprend les agents actifs incorporés dans une matrice gonflable; et
(C) un troisième système de libération contenant une troisième proportion d'agents actifs du groupe constitué par les vitamines, les minéraux et/ou les oligoéléments, qui comprend les agents actifs incorporés dans une matrice gonflable,
- dans laquelle les systèmes de libération (A), (B) et (C) libèrent chacun des agents actifs après l'application perorale de manière contrôlée et décalée dans le temps les uns par rapport aux autres,
- dans laquelle les systèmes de libération (A), (B) et (C) comprennent chacun des particules discrètes ayant des diamètres de particules dans la plage allant de 10 à 1.000 µm,
- dans laquelle les systèmes de libération (A), (B) et (C) sont réunis en une unité,
- dans laquelle les systèmes de libération (A), (B) et (C) contiennent des agents actifs au moins partiellement différents les uns des autres, et
- dans laquelle les systèmes de libération (B) et (C) contiennent différentes quantités de matériau de matrice de manière à ce que les agents actifs contenus dans les systèmes de libération (B) et (C) soient libérés de manière décalée dans le temps les uns par rapport aux autres, le rapport entre la quantité de matériau de matrice dans le système de libération (C) et la quantité de matériau de matrice dans le système de libération (B) variant dans la plage allant de 15:1 à 1,1:1.

2. Composition selon la revendication 1, **caractérisée en ce que** la matrice est gonflable lors du contact avec des milieux fluides aqueux, notamment lors du contact avec le suc gastrique et/ou intestinal.

3. Composition selon la revendication 1 ou 2, **caractérisée en ce que** la matrice comprend un polymère gonflable, notamment la cellulose ou un dérivé de cellulose, avantageusement un éther de cellulose ou un mélange de différents éthers de cellulose, de préférence une hydroxyalkylcellulose, de manière particulièrement préférée l'hydroxypropylméthylcellulose ou un mélange de méthylcellulose et d'éthers de cellulose mixtes, tels que l'hydroxypropyl-hydroxybutylméthylcellulose, ou en est constituée.

4. Composition selon une ou plusieurs des revendications précédentes, **caractérisée en ce que** la matrice libère les agents actifs après l'application perorale de manière à ce que les interactions négatives entre les agents actifs soient au moins essentiellement évitées ou au moins minimisées et/ou à ce que la matrice libère les agents actifs après l'application perorale en continu pendant une période de temps définie et/ou en quantités respectivement contrôlées, la résorption des agents actifs dans le tractus gastrique/intestinal étant ainsi optimisée, un surdosage et/ou une surcharge à court terme des systèmes de résorption étant notamment au moins essentiellement évités.

5. Composition selon une ou plusieurs des revendications précédentes, **caractérisée en ce que** les particules discrètes sont des corps moulés, de préférence des pastilles, des agglomérats, des granulats ou analogues.

6. Composition selon une ou plusieurs des revendications précédentes, **caractérisée en ce que** les particules discrètes présentent un diamètre de particule dans la plage allant de 200 à 750 µm, de préférence de 250 à 600 µm, de manière particulièrement préférée de 300 à 550 µm, de manière tout particulièrement préférée d'environ 500 µm.

7. Composition selon une ou plusieurs des revendications précédentes, **caractérisée en ce que** les particules discrètes, notamment les corps moulés, des différents systèmes de libération (A), (B) et (C) sont respectivement réunies ou compactées en pièces moulées, notamment en pièces moulées de comprimés ("mini-comprimés"), les pièces moulées présentant notamment un diamètre de 1.000 à 3.000 µm, notamment de 1.200 à 2.800 µm, de préférence de 1.500 à 2.500 µm.

8. Composition selon une ou plusieurs des revendications précédentes, **caractérisée en ce que** les différents systèmes de libération (A), (B) et (C) sont réunis en une unité sous la forme d'un comprimé, notamment d'un comprimé pelliculé, d'une gélule, d'une pilule ou d'une dragée.

9. Composition selon la revendication 7 ou 8, **caractérisée en ce que** les pièces moulées, notamment les pièces moulées de comprimés ("mini-comprimés"), sont réunies ou compactées en un comprimé total, le comprimé total présentant notamment un diamètre de 10 à 30 mm, notamment de 10 à 25 mm, et/ou le comprimé total étant notamment transformé en un comprimé pelliculé.

10. Composition selon une ou plusieurs des revendications précédentes, **caractérisée en ce que** la quantité de matériau de matrice dans le système de libération (B), par rapport au système de libération (B), est de 1 à 15 % en poids, notamment de 1 à 10 % en poids, avantageusement de 1 à 5 % en poids, de préférence de 3 à 5 % en poids, et/ou **en ce que** la quantité de matériau de matrice dans le système de libération (C), par rapport au système de libération (C), est de 1 à 40, notamment de 2 à 35 % en poids, avantageusement de 4 à 10 % en poids, de préférence de 6 à 8 % en poids.

11. Composition selon une ou plusieurs des revendications précédentes, **caractérisée en ce que** le rapport entre la quantité de matériau de matrice dans le système de libération (C) et la quantité de matériau de matrice dans le système de libération (B) varie dans la plage allant de 10:1 à 1,2:1, de préférence de 5:1 à 1,5:1, de manière particulièrement préférée de 3:1 à 1,5:1, et/ou **en ce que** le rapport entre la quantité de matériau de matrice dans le système de libération (C) et la quantité de matériau de matrice dans le système de libération (B) est supérieur à 1,2:1, notamment supérieur à 1,5:1, de préférence supérieur à 3:1, de manière particulièrement préférée supérieur à 5:1.

12. Composition selon une ou plusieurs des revendications précédentes, **caractérisée en ce que** les systèmes de libération (B) et (C) contiennent différents matériaux de matrice, de manière à ce que les agents actifs contenus dans les systèmes de libération (B) et (C) soient libérés de manière décalée dans le temps les uns par rapport aux autres.

13. Composition selon une ou plusieurs des revendications précédentes, **caractérisée en ce que** le première système de libération (A) comprend en tant qu'agents actifs des fluorures, notamment du fluorure de sodium; des sels de cuivre, notamment du sulfate de cuivre (II); des sels de zinc, notamment de l'oxyde de zinc; de la biotine; des vitamines du groupe D, notamment de la vitamine D₃; de la vitamine K₁, de la vitamine E ou ses dérivés, notamment de l'acétate de tocophérol; Q 10; de la vitamine A; des esters de lutéine; de la zéaxanthine; du nicotinamide; de la cyanocobalamine; et/ou
**en ce que** le deuxième système de libération (B) comprend en tant qu'agents actifs de l'acide ascorbique; de la riboflavine; de l'acide folique; du nitrate de thiamine; du chlorhydrate de pyridoxine; de l'iodate de potassium; du molybdate de sodium; des sels de fer, notamment du fumarate de fer (II); des sels de manganèse, notamment du sulfate de manganèse (II); du chlorure de potassium; des sels de magnésium, notamment de l'oxyde de magnésium; et/ou en ce que le troisième système de libération (C) comprend en tant qu'agents actifs des sels de sélénium, notamment du sélénite de sodium; du pantothénate de calcium; des sels de chrome, notamment du chlorure de chrome; de l'hydrogénophosphate de calcium.

14. Composition selon une ou plusieurs des revendications précédentes, **caractérisée en ce que** les systèmes de libération (A), (B) et/ou (C) contiennent également au moins un véhicule pharmaceutiquement inoffensif ou des excipients.

15. Composition selon une ou plusieurs des revendications précédentes, **caractérisée en ce que** les systèmes de libération (A), (B) et/ou (C) contiennent également au moins un agent actif et/ou composant supplémentaire, choisi dans le groupe constitué par les protéines et les concentrés de protéines; les acides aminés; les sels de glucosamine et les sels de chondroïtine, notamment les sulfates; les champignons et les extraits de champignons; les levures et les extraits de levures; les algues et les extraits d'algues; le kombucha et les extraits de kombucha; les enzymes et les coenzymes; les composants végétaux secondaires et les plantes et extraits végétaux correspondants; les cultures probiotiques et prébiotiques; les acides de fruits et leurs sels; ainsi que leurs mélanges.

16. Composition selon une ou plusieurs des revendications précédentes, **caractérisée en ce que** les systèmes de libération (A), (B) et/ou (C) contiennent également au moins un composant supplémentaire, notamment choisi dans le groupe des additifs et/ou des adjuvants, tels que les charges, les diluants, les liants, les agents mouillants, les stabilisateurs, les colorants, les agents tampons, les agents olfactifs, les agents gustatifs, les édulcorants, les aromatisants, les adjuvants d'usinage et les conservateurs, ainsi que leurs mélanges.

17. Composition selon une ou plusieurs des revendications précédentes, **caractérisée en ce que** la composition se présente sous forme solide, notamment sous la forme d'une poudre ou d'un granulat ou de préférence sous la forme d'un comprimé, notamment d'un comprimé pelliculé, d'une gélule, d'une pilule ou d'une dragée.

18. Composition selon une ou plusieurs des revendications précédentes, **caractérisée en ce que** la composition est un médicament ou une préparation pharmaceutique, un produit médical, un complément alimentaire ou un aliment diététique.
